Europäisches Patentamt

European Patent Office

Office européen des brevets

(11) Publication number: **0 110 858**
A1

# EUROPEAN PATENT APPLICATION

(21) Application number: 83870107.6

(51) Int. Cl.³: **B 01 D 53/22**, C 07 C 7/144

(22) Date of filing: 11.10.83

(30) Priority: 13.10.82 US 434136
17.02.83 US 467414

(43) Date of publication of application: 13.06.84
Bulletin 84/24

(84) Designated Contracting States: DE FR GB IT NL

(71) Applicant: Monsanto Company, Patent
Department 800 North Lindbergh Boulevard, St. Louis
Missouri 63167 (US)

(72) Inventor: Narayan, Raghu Subramani, 912 Camargo
Drive, Ballwin Missouri 63011 (US)
Inventor: Patton, Clyde Jerome, 7012 Three Bridges
Circle, Raleigh North Carolina 27612 (US)

(74) Representative: McLean, Peter et al, Monsanto Europe
S.A. Patent Department Avenue de
Tervuren 270-272 Letter Box No 1, B-1150 Brussels (BE)

(54) Membrane gas separation process.

(57) A process for separating methane from a gaseous mixture (1) of carbon dioxide and methane wherein the gas mixture is brought into contact with a first separator (7) having a membrane (8) more permeable to carbon dioxide than methane to allow a carbon dioxide-enriched gas (2) to permeate the membrane. The non-permeated gas (6) is passed into contact with at least one subsequent separator (9) from which a carbon dioxide-enriched permeate gas (4) is withdrawn as a recycle stream (2).

## MEMBRANE GAS SEPARATION PROCESS
## BACKGROUND OF THE INVENTION

This invention relates to the method of separating gases by means of membranes selective for the permeation of one or more gases of a mixture of gases. More particularly, the invention relates to a method for the selective membrane separation of acid gases, such as carbon dioxide and/or hydrogen sulfide, from hydrocarbon gas, such as methane, by gaseous diffusion or permeation wherein the separating efficiency of a gaseous separation membrane stage is improved without altering the characteristics of the membranes employed.

Gas separation membranes have been developed in recent years for separating many gases from mixtures thereof with many other gases and vapors. The separation of gases with membranes, whether by diffusion or permeation through the material of the membrane, depends to a large extent on the differential pressure maintained on either side of the membrane. The pressure considerations differ considerably from those required when liquid materials are to be separated by means of semi-permeable membranes.

Generally the flux of one or more gases through a semi-permeable membrane is enhanced as the pressure differential at the two sides of the membrane is increased. However, practical limits to such increased pressures are always imposed, generally by the strength of the membranes employed, whether in flat film or hollow fiber form. In addition, the cost of compressing gases and gaseous mixtures through repeated stages of separation such as cascaded separation steps or stages rapidly becomes economically limiting. Such cascaded separation steps or stages are illustrated for instance in U.S. Patents 2,540,152; 2,617,493; 3,713,271; 4,104,037 and 4,312,755; and also in European Patent Publication Number 0,029,600 A1.

In particular U.S. Patent 4,119,417 discloses a pair of membrane separators connected in series and having a number of piping configurations for feeding both permeated and non-permeated gases from the two separators to other points in the systems. The membranes used in this gas separator system apparently each have a separation factor of about 4 or 5 and the patentee is attempting to obtain a higher separation factor by staging these separators with gas recompression between stages.

A fast permeating gas such as hydrogen can be effectively recovered from a gas mixture containing slower permeating gases in a single stage by membrane gas separation where the membrane exhibits high selectivity to the permeability of hydrogen as compared to the permeability of slower gases. In this regard see for instance U.S. 4,180,553 and U.S. 4,172,885. See also U.S. 4,180,552 where the separation is achieved with two permeator stages in series. Although hydrogen can be recovered at relatively high purities and high recovery rates, the non-permeating gas is often not at high purities for any component. U.S. 4,130,403 discloses the use of a pair of separators in series where the low pressure permeate gas from the second separator is recompressed to feed a third separator, the non-permeate gas of which is recycled to the stream bridging the pair of separators in series. The undue amount of separation and compression stages are generally economically unattractive.

A simple effective and economic method to separate gases at relatively high levels of recovery and purity for both a permeating and non-permeating species without increasing differential pressures across the membrane would be advantageous and desirable.

In describing the present invention a particulary convenient analytical characteristic of polymeric gas permeable membranes includes the permeability of the membrane for a specific gas through the membrane. The permeability ($P_a/\ell$) of a membrane for gas "a" of a gas mixture through a membrane of thickness "$\ell$" is the volume of gas, referred to standard temperature and pressure (STP), which passes through the membrane per unit of surface area of membrane, per unit of time, per unit of differential partial pressure of the permeating species across the thickness of the membrane. One method for expressing permeabilities is cubic centimeters (STP) per square centimeter of membrane area per second per differential partial pressure of 1 centimeter of mercury across the membrane thickness ($cm^3(STP)/cm^2$-sec-cmHg). Unless otherwise noted, all permeabilities are reported herein at temperatures and pressure of 15°C and 1 atmosphere, respectively. The permeabilities are reported in gas permeation units (GPU), which are $cm^3(STP)/cm^2$-sec-cmHg x $10^6$; thus 1 GPU is 1 x $10^6 cm^3(STP)/cm^2$-sec-cmHg. Another convenient relationship for expressing gas permeation characteristics of membranes is separation factor. A separation factor, $\propto$ a/b, for a membrane for a given pair of gases "a" and "b" is defined as the ratio of the permeability ($P_a/\ell$) of a membrane of thickness "$\ell$" for a gas "a" of a gas mixture to the permeability ($P_b/\ell$) of the same membrane to gas "b".

In practice, separation factor with respect to a given pair of gases for a given membrane can be determined by employing numerous techniques which provide sufficient information for calculation of permeabilities for each of the gases. Several of the many techniques available for determining permeabilities and separation factors are disclosed by Hwang et.al., Techniques of Chemistry, Volume VII, Membranes in Separations, John Wiley & Sons, 1974 at Chapter 13, pages 296-322.

Measurements can be made for pure gas permeation or for blend gas permeation. However, experience has shown that the measured permeability of a membrane for a gas species is higher for pure gas permeation than for blend gas permeation. In general, it is more desirable to determine gas permeation characteristics for blend gases, since the permeabilities and separation factors more closely predict actual membrane gas separation performance characteristics.

There has been discovered a method for separating gases which increases the recovery of the non-permeating product gas or gases that does not require repeated gas recompression between stages of separation such as in cascaded separation schemes. This method also does not require the use of a second separator stage to further separate the components of the recycle stream. This novel method is applicable to any separation of gaseous mixtures which are separable by permeation or diffusion through membranes, but in which previously known applications of a single permeator stage will not produce the desired recovery of non-permeated product gas or gases. This method is especially advantageous where the membrane utilized exhibits a moderate separation factor for at least two·gases where it is desired that one of the gases be concentrated in a permeated product gas and the other gas be concentrated in a non-permeated product gas. Separation factor of the

membrane, designated as $\alpha_j^i$, is the ratio of the permeability of the membrane for a faster permeating gas species (species i) to the permeability of the membrane for a slower permeating gas species (species j). Such moderate separation factors range from about 8 to about 30.

The novel method of the present invention is particularly advantageous to the separation of acid gases, for instance carbon dioxide and/or hydrogen sulfide, from a gaseous mixture containing acid gases and hydrocarbon gas, for instance such as methane. Preferably the membrane will exhibit a separation factor for carbon dioxide to methane, $\alpha\frac{CO_2}{CH_4}$, of at least about 8.

## SUMMARY OF THE INVENTION

This invention provides a membrane gas separation process for recovering a non-permeate gas enriched in at least one gas from a mixture comprising said at least one gas and at least one other gas said process comprising (a) providing said mixture at a permeating pressure to at least two first separators having membranes exhibiting selectivity to the permeation of said at least one other gas compared to the permeation of said at least one, other gas compared to the permeation of said at least one gas, wherein said first separators have a determined first separator membrane surface area; (b) allowing a non-permeate gas from said first separator to pass to at least two subsequent separators having membranes exhibiting selectivity to the permeation of said at least one other gas compared to the permeation of said at least one gas, wherein said subsequent separators have a determined subsequent separator membrane surface area, the ratio of said determined first separator membrane surface area to said determined subsequent separator membrane surface area being at least 0.3; (c) withdrawing a permeate gas enriched in said at least one other gas from said first separators; (d) withdrawing a non-permeate gas enriched in said at least one gas from said subsequent separators; and (e) withdrawing a recycle permeate gas from said subsequent separators wherein at least part of said recycle permeate gas is returned to said mixture, thereby enhancing the volume of said at least one gas recovered in said non-permeate gas. This process is particularly advantageous for use with separators having membranes exhibiting selectivity to the permeation of the acid gas component, for instance carbon dioxide and/or hydrogen sulfide, and where the membrane exhibits a separation factor for carbon dioxide to methane, $\alpha\frac{CO_2}{CH_4}$, of at least 8, preferably in the range of 8 to 30.

The process is useful where the hydrocarbon gas of the mixture is a lower hydrocarbon gas, such as methane, ethane, propane, butane, isobutane, pentane and isopentane, including mixtures thereof. The process is also particularly useful where the hydrocarbon gas consists of methane. A preferred application of the process is for separating mixtures of a recycled permeate gas and a feed gas where the feed gas comprises from about 30 to about 70 mole percent methane and from about 30 to about 70 mole percent carbon dioxide. The process is particularly advantageous where the methane-enriched non-permeate gas comprises at least about 90 mole percent methane and at least 90 percent of the total methane of the feed gas.

This process is particularly useful where the membranes exhibit selectivity to the permeation of said at least one other gas compared to the permeation of said at least one gas by a factor in the range of 8 to 30. That is, the membranes will exhibit a separation factor, α, for the gases in the range of 8 to 80.

Often it is desired that in such process said at least one first separator comprises a plurality of separators having a determined first separator membrane surface area and said at least one subsequent separator comprises a plurality of separators having a determined subsequent separator membrane surface area and the ratio of said determined first separator membrane surface area to said determined subsequent separator surface area be at least 0.3.

A further aspect of this invention is membrane gas separation apparatus comprising at least two first separators having membranes exhibiting selectivity to the permeation of one gas compared to the permeation of at least one other gas, at least two subsequent separators having membranes exhibiting selectivity to the permeation of said one gas compared to the permeation of said at least one other gas, means for providing a mixture comprising said at least one gas and said at least one other gas at a permeating pressure to said first separators, means for allowing a non-permeate gas to flow from said first separators to said subsequent separators, means for withdrawing a permeate gas enriched in said at least one gas from said first separators, means for withdrawing a non-permeate gas enriched in said at least one other gas from said subsequent separators, means for withdrawing a recycle permeate gas from said subsequent separators, and means for returning at least part of said recycle permeate gas to said means for providing a mixture.,

In a preferred aspect of the apparatus of this invention said first separators have a determined first-separator, membrane surface area and said subsequent separators have a determined subsequent-separator, membrane surface area, the ratio of said first-separator membrane surface area to said subsequent-separator membrane surface area being at least 0.3.

A still further preferred aspect of this invention is a process for separating acid gas, such as carbon dioxide and/or hydrogen sulfide, from methane wherein a gaseous mixture comprising carbon dioxide and/or hydrogen sulfide and methane is brought into contact with one side of a membrane more permeable to carbon dioxide than to methane to allow the acid gas to permeate the membrane to the other side thereof. Permeated gases from the other side of the membrane are withdrawn and split into a recovery stream and a recycle stream, said recycle stream being returned into contact with the one side of the membrane. The membrane has a separation factor of at least about 8 for carbon dioxide over methane.

BRIEF DESCRIPTION OF THE DRAWINGS

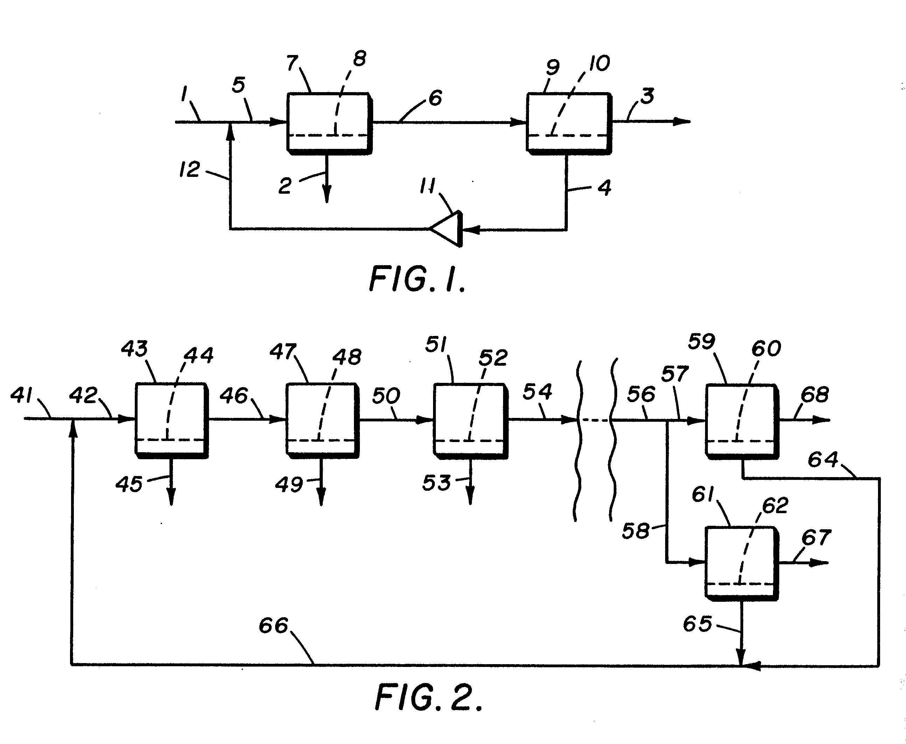

Figures 1, 2, 3 and 4 are schematic representations of apparatus utilized in carrying out aspects of the process of the present invention.

DETAILED DESCRIPTION OF THE INVENTION

Referring now in detail to the drawings, Figure 1 schematically illustrates an apparatus useful for carrying out an aspect of the process of the present invention. The feed gas comprising a hydrocarbon gas, such as methane, and an acid gas, such as carbon dioxide, is fed through a line 1 where it mixes with a recycle permeate gas in line 12 forming a mixture which·is fed through line 5 to a first separator 7 having a membrane 8 which exhibits selectivity to the permeation of carbon dioxide compared to the permeation of methane.

The mixture is at a permeating pressure which allows a carbon dioxide-enriched gas to selectively permeate the membrane 8. As a carbon dioxide-enriched permeate gas is withdrawn at a reduced pressure from the first separator 7 through a line 2, a non-permeate gas is allowed to pass through line 6 from the first permeator 7 to a subsequent permeator 9 having a membrane 10 exhibiting selectivity to the permeation of carbon dioxide compared to the permeation of methane. Carbon dioxide also selectively permeates a membrane 10 producing a methane-enriched non-permeate gas which is withdrawn at a reduced pressure from subsequent separator 9 through line 3. The permeate gas from subsequent separator 9 is allowed to pass through line 4 to a compressor 11 to be fed back at a permeating pressure through line 12 as a recycle permeate gas.

The membranes 8 and 10 exhibit selectivity to the permeation of acid gas component, for instance carbon dioxide, as compared to the permeation of the hydrocarbon gas, for instance methane, accordingly these membranes are characterized as having a separation factor for carbon dioxide over methane of at least about 8. The separation factor is represented as $\alpha\frac{CO_2}{CH_4}$. The membranes 8 or 10 may be in the form of a flat sheet, but preferably are in the form of polymeric hollow fibers. Generally a membrane will consist of a bundle of a plurality of hollow fiber membranes. Among the preferred hollow fiber membranes are multicomponent membranes as disclosed in U.S. Patent 4,230,463. A variety of useful separator designs include those disclosed in U.S. Patents 3,616,928; 3,339,341; 3,422,008; 3,528,553; 3,702,658; and 4,315,819.

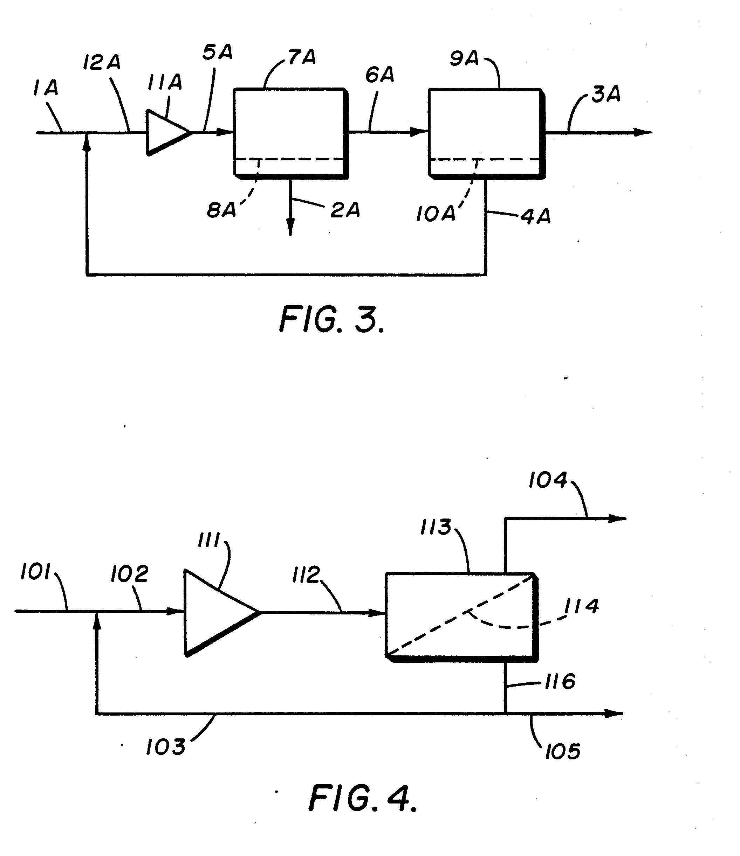

An alternative arrangement is disclosed in Figure 3, where the feed gas is supplied at a low pressure, requiring compression to a permeating pressure. The permeate gas from subsequent separator 9A is allowed to pass through line 4A as a recycle permeate gas which mixes with the low pressure feed gas flowing in line 1A. They form a low pressure gas mixture which flows through line 12A to compressor 11A which provides the mixture at a permeating pressure.

Operation as shown in Figure 3 is advantageous since both the recycle permeate gas and the feed gas are compressed to a permeating pressure by the compressor. This allows considerable savings in capital investment, installation cost and operating cost.

It is understood, of course, that separators 7 and 9 of Figure 1 and 7A and 9A of Figure 3 represent a single separator or a plurality of separators depending on the membrane surface area required for the desired separation and the most economical manner of providing membrane surface area in a separator.

Referring now to Figure 2 there is shown schematic representation of alternative apparatus for carrying out another aspect of the process of the present invention. A gas mixture comprising, for instance, methane and carbon dioxide is supplied through line 42 and comprises a feed gas supplied through line 41 and a recycle permeate gas supplied through line 66. The gas mixture is provided at a permeating pressure to a plurality of first separators 43, 47 and 51 in series. Each first separator has a membrane 44, 48 and 52 which exhibits selectivity to the permeation of carbon dioxide such that a carbon dioxide-enriched permeate gas can be withdrawn from the separators through lines 45, 49 and 53. The non-permeate gas is allowed to pass to each first separator in series with a minimal drop in permeating pressure for instance through lines 46, 50 and 54. The number of first separators in series depends upon such factors as the total gas flow rate, the desired separation and the economical amount of membrane surface area that can be provided in a separator. The non-permeate gas from the plurality of first separators is supplied to a plurality of subsequent separators through line 56 which divides the non-permeate gas into separate gas streams which are fed, for instance, through lines 57 and 58 to subsequent separators 59 and 61 which are arranged in parallel. Such subsequent separators 59 and 61 have membranes 60 and 62 respectively which exhibit selectivity to the permeation of carbon dioxide thereby allowing a permeate gas more enriched in carbon dioxide as compared to the gas supplied to the subsequent separators to be withdrawn through lines 64 and 65 respectively. The permeate gas streams can be combined as a recycle permeate gas and transmitted through line 66 for blending with the feed gas in line 41. The non-permeate gas from the subsequent separators will be substantially enriched in methane and can be withdrawn from the subsequent separators through lines 68 and 67. The number of subsequent separators also depends on such variables as the amount of gas to be processed, the desired separation and the amount of membrane surface area which can be provided within a separator.

Depending on the separation to be effected the arrangement of the first separators, for instance 43, 47 and 51, may be in series as illustrated in Figure 2 or in parallel or in a combination series/parallel arrangement. Likewise the arrangement of the subsequent separators, for instance 59 and 61, may be in parallel as illustrated in Figure 2 or in series or in a combination series/parallel arrangement.

Referring now to Figure 4, there is shown schematically apparatus useful for carrying out an aspect of the process of the present invention with a single membrane separator. A gaseous mixture, for instance of carbon dioxide and methane, is fed by a compressor 111 through a line 112 into a separator 113 having a membrane 114 more permeable to carbon dioxide than to methane.

Carbon dioxide selectively permeates the membrane 114 from one side to the other and is withdrawn from the separator 113 through a line 116 as a permeate gas stream enriched in carbon dioxide. A non-permeate gas is withdrawn from the separator 113 through a line 104; this stream of gas is a methane-enriched recovery stream. A portion of the carbon dioxide enriched permeate gas stream passing through line 116 is withdrawn from this line and recycled or returned through a line 103 to the suction side of the compressor 111 to be fed back into contact with the one side of the membrane 114. The remainder of the permeate gas stream is withdrawn as a carbon dioxide enriched recovery stream 105. The amount of the carbon dioxide enriched permeate gas stream to be returned to the inlet side of the separator 113 is about 10-50 percent.

As a result of extensive membrane gas separation operations a considerable body of membrane permeation data has been accumulated which assures that computer assistance simulations of membrane gas separations can be utilized to predict membrane gas separator performance with reasonable accuracy. Such membrane permeation data includes, for instance, when a feed gas comprising 21.8 mole percent carbon dioxide and 76.9 mole percent methane was contacted with a gas separation membrane more permeable to carbon dioxide a permeate gas comprising 78.6 mole percent carbon dioxide was withdrawn. The non-permeate gas was enriched to 79.0 mole percent methane. When the same membrane was contacted with a feed gas comprising substantially more carbon dioxide at 79.8 mole percent carbon dioxide and only 16.9 mole percent methane and operated under similar conditions, of temperature, pressure and flow rate the permeate gas was enriched to 94.4 mole percent percent carbon dioxide. However the non-permeate was enriched to only 19.2 mole percent methane.

The following examples are based on computer simulations of a membrane gas separation of a gas mixture comprising carbon dioxide and methane. Metric units of measure used in the following examples are based on the following conversion factors for units of measure commonly used in the United States.

$$SCFH \times 0.0268 = N\ m^3/hr\ ,$$
$$psi \times 6.89\quad = kPa,$$
$$ft^2 \times 0.0929\quad = m^2\ ,\ and\ ,$$
$$BHP \times 0.7457\quad = kW;$$

where SCFH is a measure of flow in standard cubic feet per hour,

$Nm^3/hr$ is a measure of flow in normal cubic meters per hour,

psi is a measure of pressure in pounds force per square inch,

kPa is a measure of pressure in kilopascals,

$ft^2$ is a measure of area in square feet,

$m^2$ is a measure of area in square meters,

BHP is a measure of power in brake horsepower, and

kW  is a measure of power in kilowatts.

### EXAMPLE 1

A computer simulation of a known membrane gas separation is illustrated in this example which demonstrates the detrimental effect of incremental membrane surface area on methane recovery by a membrane gas separation process.  This effect is that as membrane surface area increases, the purity of methane in a non-permeate gas can be increased but with significant dimunition of recovery of methane.  A feed gas comprising 44.0 mole percent carbon dioxide, 55.0 mole percent methane and 1.0 mole percent nitrogen is provided at a flow rate of 1115 $Nm^3$/hr. The gas is provided at a permeating pressure of 3620 kPa and supplied to a separator having 1106 square meters membrane surface area.  The permeate side of the membrane is maintained at 172 kPa.

The membranes of both separators are characterized as having a permeability for carbon dioxide of 35.0 GPU (as defined above), a permeability for methane of 2.5 GPU and a permeability for nitrogen of 1.9 GPU.  Based on the above permeabilities for carbon dioxide and methane the membrane exhibits a selectivity to the permeation of carbon dioxide compared to the permeation of methane of 14, that is the membrane has a separation factor, $\alpha\frac{CO_2}{CH_4}$ of 14.

A carbon dioxide enriched gas will selectively permeate through the membrane.  It is predicted that the permeate recovered gas will have a flow rate of 704 $Nm^3$/hr at a composition of 69.1 mole percent carbon dioxide and 30.4 mole percent methane. In this permeate gas stream 99.1 percent of the total carbon dioxide contained in the feed gas can be recovered.

It is also predicted that the non-permeate gas from the permeator will be at a flow rate of 413 $Nm^3$/hr at a composition of 1.1 mole percent carbon dioxide and 96.9 mole percent methane. In this non-permeate gas stream 65.1 percent of the total methane contained in the feed gas will be recovered.

The simulation is repeated with identical conditions except that the membrane surface area is increased to 1236 square meters. As membrane surface area is increased, the non-permeate gas becomes more enriched in methane to a level of 97.6 mole percent methane. However, the recovery of methane in the non-permeate gas is significantly reduced such that the non-permeate gas stream will comprise 60.1 percent of the total methane of the feed gas.

The simulation is further repeated with identical conditions except that the membrane surface area is increased to 1384 square meters. It is predicted that, with this further increase in membrane surface area, the non-permeate gas will become more enriched in methane to a level of 97.8 mole percent. However, the recovery of methane in the non-permeate gas is significantly further reduced such that the non-permeate gas stream will comprise only 54.9 percent of the total methane of the feed gas.

The pertinent data of this simulation is summarized in Table I.

### TABLE I

Feed Gas

Flow Rate ($Nm^3$/hr):

1115

Composition (mole percent):

CO$_2$: · 44.0

CH$_4$: 55.0

N$_2$: 1.0

Membrane Area (m$^2$):

|      | 1106 | 1236 | 1384 |

Non-Permeate Recovered Gas:

Flow Rate (Nm$^3$/hr):

|      | 413 | 378 | 346 |

Composition (mole percent):

| | | | |
|---|---|---|---|
| $CH_4$ | 96.9 | 97.6 | 97.8 |
| $CO_2$ | 1.1 | 0.4 | 0.1 |
| $N_2$ | 2.0 | 2.0 | 2.1 |

$CH_4$ Recovery

$$\frac{CH_4 \text{ in Non-Permeate}}{CH_4 \text{ in Feed}} \times 100$$

|      | 65.1 | 60.1 | 54.9 |

The following examples 2 through 5 illustrate the process of the present invention wherein the recovery of methane in the non-permeate gas is significantly enhanced although there is an increase in total membrane surface area.

### EXAMPLE 2

A computer simulation of a membrane gas separation is illustrated in this example utilizing a feed gas comprising 44.0 mole percent carbon dioxide, 55.0 mole percent methane and 1.0 mole percent nitrogen provided at a flow rate of 1115 Nm$^3$/hr. The gas is provided at a permeating pressure of 3620 kPa. The feed gas is combined with a permeate recycle gas also compressed to 3620 kPa and supplied to a first separator having 743 square meters of membrane surface area. A non-permeate gas from the first separator is allowed to pass to a subsequent separator having 362 square meters of membrane surface area.

The membranes of both separators are characterized as having a permeability for carbon dioxide of 35.0 GPU (as defined above), a permeability for methane of 2.5 GPU and a permeability for nitrogen of 1.9 GPU. Based on the above permeabilities for carbon dioxide and methane the membrane exhibits a selectivity to the permeation of carbon dioxide compared to the permeation of methane of 14, that is the membrane has a separation factor, $\alpha\frac{CO_2}{CH_4}$ of 14.

It is predicted that a permeate recycle gas having a composition of 30.9 percent carbon dioxide and 68.1 percent methane can be withdrawn from the subsequent separator at a flow rate of 115 $Nm^3$/hr and returned as permeate recycle gas to be mixed with the feed gas.

The permeate gas from the first separator is predicted to be at a flow rate of 617 $Nm^3$/hr at a composition of 78.1 mole percent carbon dioxide and 21.6 mole percent methane. This permeate gas stream will contain 97.9 percent of the carbon dioxide contained in the feed gas.

It is also predicted that the non-permeate gas from the subsequent permeator will be at a flow rate of 499 $Nm^3$/hr at a composition of 2.0 mole percent carbon dioxide and 96.1 mole percent methane. This non-permeate gas stream will recover 78.2 percent of the total methane supplied in the feed gas.

The permeate gas streams will flow from each permeator at a pressure of 172 kPa. In order to compress the permeate recycle gas to the permeating gas pressure of 3620 kPa, 23 kW of power for compression will be required.

The pertinent data of the computer simulation of the Example 2 are summarized in Table II.

### EXAMPLE 3

This example further illustrates a computer simulation of a membrane gas separation of a gas mixture comprising carbon dioxide and methane. The same feed gas as utilized in the simulation of Example 2 can be separated by the process of this invention where the first permeator comprises 539 square meters of membrane surface area and the subsequent permeator comprises 697 square meters of membrane surface area.

Other operating parameters are the same as in Example 1 except that the permeate recycle gas will be at a flow rate of 300 $Nm^3$/hr with a composition of 50.1 mole percent carbon dioxide and 49.2 mole percent methane. This higher volume of recycle gas requires 60 kW of power for compression from 172 kPa to 3620 kPa.

The permeate gas recovered from the first separator is predicted to flow at a rate of 566 $Nm^3$/hr with a composition of 84.6 mole percent carbon dioxide and 15.2 mole percent methane. This permeate gas stream is predicted to contain 97.3 percent of the total carbon dioxide of the feed gas.

The non-permeate gas from the subsequent separator is predicted to flow at a rate of 550 $Nm^3$/hr with a composition of 2.0 mole percent carbon dioxide and 96.2 mole percent methane. The recovery of methane in this non-permeate gas is predicted to be significantly higher at 85.7 percent of the total methane of the feed gas.

The pertinent data of the computer simulation of this Example 3 are summarized in Table II.

### EXAMPLE 4

This example further illustrates a computer simulation of a membrane gas separation of a gas mixture comprising carbon dioxide and methane. In this example the amount of membrane surface area in the first separator is further reduced, and the amount of membrane surface area in the subsequent separator is further increased. This allows the apparatus to accommodate a higher volume of permeate recycle gas thereby enhancing the recovery of methane in the non-permeate gas stream recovered from the subsequent permeator.

The same feed gas as utilized in the simulation of Example 2 is separated by the process of this invention where the first separator comprises 427 square meters of membrane surface area and the subsequent separator comprises 957 square meters of membrane surface area. Other operating parameters are the same as in Example 2 except that 520 $Nm^3$/hr of recycle permeate gas is returned to mix with the feed gas. This higher volume of recycle permeate gas requires 102 kW of power for compression from 172 kPa to 3620 kPa.

The recovery of methane in the non-permeate gas from the subsequent separator is predicted to be increased to 90.5 percent of the total methane to the feed gas.

The pertinent data of the computer simulation of this Example 4 are summarized in Table II.

## EXAMPLE 5

This example further illustrates a computer simulation of a membrane gas separation of a gas mixture comprising carbon dioxide and methane. This example illustrates an even further enhancement in the recovery of methane in the non-permeate gas from the subsequent separator. This is achieved by further adjusting the membrane surface area among the separators and further increasing the flow rate of recycle permeate gas. The first separator comprises 279 square meters of membrane surface area and the subsequent separator comprises 1551 square meters of membrane surface area.

Other operating parameters are the same as in Example 2 except that 1400 $Nm^3$/hr of permeate

recycle gas is returned for mixture with the feed gas. This volume of recycle gas required 275 kW of power for compression from 172 kPa to 3620 kPa.

The recovery of methane in the non-permeate gas from the subsequent separator is predicted to be increaesd to 96.4 percent of the total methane of the feed gas.

The pertinent data of the computer simulation of this Example 5 are summarized in Table II.

The above Examples 2-5 illustrate aspects of the process of the present invention for recovering a hydrocarbon-enriched non-permeate gas from a mixture comprising a hydrocarbon gas and an acid gas. These examples illustrate the wide range of variables that can be manipulated to most effectively operate the process depending on feed gas conditions, membrane surface area available, .operating costs and desired separations.

This invention provides a membrane gas separation process for enhancing the recovery of a hydrocarbon gas from a mixture of hydrocarbon gas and an acid gas where as membrane surface area is increased, a recycle gas stream is utilized so that hydrocarbon recovery does not diminish but is significantly increased to higher levels.

0110858
36-52(6324)A

TABLE II

| | EXAMPLE | | | |
|---|---|---|---|---|
| | 2 | 3 | 4 | 5 |
| **Feed Gas** | | | | |
| (Stream No. 1)* | | | | |
|   Flow Rate ($Nm^3$/hr): | 1115 | 1115 | 1115 | 1115 |
|   Composition (mole percent): | | | | |
|     $CO_2$ | 44.0. | 44.0 | 44.0 | 44.0 |
|     $CH_4$ | 55.0 | 55.0 | 55.0 | 55.0 |
|     $N_2$ | 1.0 | 1.0 | 1.0 | 1.0 |
| **Permeate Recovered Gas** | | | | |
| (Stream No. 2)* | | | | |
|   Flow Rate ($Nm^3$/hr): | 617 | 566 | 539 | 501 |
|   Composition (mole percent): | | | | |
|     $CO_2$ | 78.1 | 84.6 | 88.8 | 94.8 |
|     $CH_4$ | 21.6 | 15.2 | 11.1 | 5.2 |
|     $N_2$ | .3 | .2 | .1 | nil |
| **Non-Permeate Recovered-Gas** | | | | |
| (Stream No. 3)* | | | | |
|   Flow Rate ($Nm^3$/hr): | 499 | 550 | 576 | 609 |
|   Composition (mole percent): | | | | |
|     $CO_2$ | 2.0 | 2.0 | 2.0 | 2.0 |
|     $CH_4$ | 96.1 | 96.2 | 96.2 | 96.2 |
|     $N_2$ | .9 | .8 | .8 | .8 |

TABLE II (continued)

EXAMPLE

| | 2 | 3 | 4 | 5 |
|---|---|---|---|---|
| Permeate Recycle Gas | | | | |
| (Stream No. 4)* | | | | |
| Flow Rate ($Nm^3/hr$): | 115 | 300 | 520 | 1400 |
| Composition (mole percent): | | | | |
| $CO_2$ | 30.9 | 50.1 | 62.4 | 80.4 |
| $CH_4$ | 68.1 | 49.2 | 37.0 | 19.4 |
| $N_2$ | 1.0 | .7 | .6 | .2 |
| $CO_2$ Recovery (in Stream No. 2) | 97.9 | 97.3 | 97.7 | 96.9 |
| $CH_4$ Recovery (in Stream No. 3) | 78.2 | 85.7 | 90.5 | 95.4 |
| Membrane Area | | | | |
| ($m^2$) | | | | |
| First Separator: | 743 | 539 | 427 | 279 |
| Subsequent Separator: | 362 | 697 | 957 | 1551 |
| Total: | 1105 | 1236 | 1384 | 1830 |
| Recycle Compression | | | | |
| (for Stream No. 4) | | | | |
| (Power in kW) | 23 | 60 | 102 | 275 |

*Stream number corresponds to streams in lines identified by the same
number in Figure 1.

36-52(6324)A

## EXAMPLE 6

This example illustrates a computer simulation of a membrane gas separation according to this invention using a single membrane separator. The simulation summarized in Table III predicts that feed gas of 55.0 mole percent methane and 45.0 mole percent carbon dioxide can be separated into a non-permeate gas stream of 95.0 mole percent methane and a permeate gas stream of 78.0 mole percent carbon dioxide. The non-permeate gas stream will recover 78.0 percent of the total methane in the feed gas. None of the permeate gas stream is recycled. The pressure at the feed side of the membrane is 2520 kPa; the pressure at the permeate side of the membrane is 172 kPa.

### TABLE III

| Stream No.* | 101 | 102 | 103 | 104 | 105 |
|---|---|---|---|---|---|
| Flow Rate: | | | | | |
| $(10^3 \ Nm^3/hr)$ | 5.6 | 5.6 | 0 | 2.6 | 3.0 |
| Composition: | | | | | |
| $CH_4$ (mol %) | 55.0 | 55.0 | - | 95.0 | 22.0 |
| $CO_2$ (mol %) | 45.0 | 45.0 | - | 5.0 | 78.0 |

* Stream number corresponds to streams in lines identified by the same number in Figure 4.

## EXAMPLE 7

This example illustrates a computer simulation of a membrane gas separation similar to that of Example 6 except that 25.7 percent of the permeate gas stream is recycled to the gaseous mixture. The simulation summarized in Table II predicts that the feed gas can be separated into a non-permeate gas stream of 95.0 mole percent methane and a permeate gas stream of 80.8 mole percent carbon dioxide. The non-permeate gas stream will recover 81.5 percent of the total methane in the feed gas.

TABLE IV

| Stream No.* | 101 | 102 | 103 | 104 | 105 |
|---|---|---|---|---|---|
| Flow Rate: | | | | | |
| ($10^3$ $Nm^3$/hr) | 5.6 | 6.6 | 1.0 | 2.6 | 3.0 |
| Composition: | | | | | |
| $CH_4$ (mol %) | 55.0 | 49.6 | 19.2 | 95.0 | 19.2 |
| $CO_2$ (mol %) | 45.0 | 50.4 | 80.8 | 5.0 | 80.8 |

* Stream number corresponds to streams in lines
  identified by the same number in Figure 4.

### EXAMPLE 8

This example illustrates a computer
simulation of a membrane gas separation similar to
that of Example 7 except that the 50 percent of the
permeate gas stream is recycled to the gaseous
mixture. The simulation summarized in Table V
predicts that the feed gas can be separated into a
non-permeate gas stream of 95.0 mole percent methane
and a permeate gas stream of 84.5 mole percent carbon
dioxide. The non-permeate gas stream will recover
85.3 percent of the total methane in the feed gas.

TABLE V

| Stream No.* | 101 | 102 | 103 | 104 | 105 |
|---|---|---|---|---|---|
| Flow Rate: | | | | | |
| ($10^3$ $Nm^3$/hr) | 5.6 | 8.4 | 2.8 | 2.8 | 2.8 |
| Composition: | | | | | |
| $CH_4$ (mol %) | 55.0 | 41.7 | 15.5 | 95.0 | 15.5 |
| $CO_2$ (mol %) | 45.0 | 58.3 | 84.5 | 5.0 | 84.5 |

* Stream number corresponds to streams in lines
  identified by the same number in Figure 4.

Membranes suitable for use in this process
often will have substantially as large a separation
factor for hydrogen sulfide over methane as for carbon
dioxide over methane, i.e., at least about 9. Thus,
hydrogen sulfide and mixtures of hydrogen sulfide and carbon dioxide can be separated from methane with substantially the same efficiency as that obtained in separating carbon dioxide from methane.

While the invention has been described herein with regard to certain specific embodiments, it is not so limited.  It is to be understood that variations and modifications thereof may be made by those skilled in the art without departing from the spirit and scope of the invention.

CLAIMS:

1. A membrane gas separation process for recovering a non-permeate gas enriched in at least one gas from a mixture comprising said at least one gas and at least one other gas said process comprising

    (a)  providing said mixture at a permeating pressure to at least two first separators having membranes exhibiting selectivity to the permeation of said at least one other gas compared to the permeation of said at least one other gas compared to the permeation of said at least one gas, wherein said first separators have a determined first separator membrane surface area;

    (b)  allowing a non-permeate gas from said first separator to pass to at least two subsequent separators having membranes exhibiting selectivity to the permeation of said at least one other gas compared to the permeation of said at least one gas, wherein said subsequent separators have a determined subsequent separator membrane surface area, the ratio of said determined first separator membrane surface area to said determined subsequent separator membrane surface area being at least 0.3;

    (c)  withdrawing a permeate gas enriched in said at least one other gas from said first separators;

    (d)  withdrawing a non-permeate gas enriched in said at least one gas from said subsequent separators; and

(e)　withdrawing a recycle permeate gas from said subsequent separators wherein at least part of said recycle permeate gas is returned to said mixture, thereby enhancing the volume of said at least one gas recovered in said non-permeate gas.

2.　The process of claim 1 wherein said at least one gas comprises a hydrocarbon gas and said at least one other gas comprises an acid gas.

3.　The process of claim 2 wherein the membranes exhibiting selectivity to the permeation of said at least one other gas compared to the permeation of said at least one gas have a separator factor, $\alpha$, in the range of 8 to 30.

4.　The process of claim 3 wherein said hydrocarbon gas comprises methane.

5.　The process of claim 4 wherein said acid gas comprises carbon dioxide.

6.　The process of claim 5 wherein said mixture consists of said recycle permeate gas and a feed gas comprising from about 30 to about 70 mole percent methane and from about 30 to about 70 mole percent carbon dioxide.

7.　The process of claim 6 wherein said non-permeate gas enriched in said at least one gas comprises at least 90 mole percent methane and at least 90 percent of the total methane of said feed gas.

8.　A process for separating an acid gas from a gas mixture comprising a hydrocarbon gas, said process comprising

(a)　feeding said gas mixture into contact with one side of a membrane more permeable to acid gas than to hydrocarbon gas such that acid gas selectively permeates to the other side of the membrane, said membrane having a separation factor of at least about 8 for carbon dioxide over methane;

(b) withdrawing a non-permeate gas stream from said one side of said membrane;

(c) withdrawing a permeate gas stream from said other side of the membrane; and

(d) returning a portion of said permeate gas stream to said gaseous mixture as a recycle stream.

9. The process of claim 8 wherein 10 to 50 percent of said permeate gas stream is returned to said gaseous mixture.

10. The process of claim 9 wherein said gaseous mixture consists of a feed gas comprising from 20 to 80 volume percent methane and said recycle stream.

11. The process of claim 10 wherein the non-permeate gas stream comprises at least 90 volume percent methane.

12. The process of claim 11 wherein a sufficient amount of said permeate gas stream is returned as a recycle stream such that said non-permeate gas stream contains at least 80 percent of the methane in said feed gas.

13. Membrane gas separation apparatus comprising

at least two first separators having membranes exhibiting selectivity to the permeation of one gas compared to the permeation of at least one other gas,

at least two subsequent separators having membranes exhibiting selectivity to the permeation of said one gas compared to the permeation of said at least one other gas,

means for providing a mixture comprising said at least one gas and said at least one other gas at a permeating pressure to said first separators,

means for allowing a non-permeate gas to flow from said first separators to said subsequent separators,

means for withdrawing a permeate gas enriched in said at least one gas from said first separators,

means for withdrawing a non-permeate gas enriched in said at least one other gas from said subsequent separators,

means for withdrawing a recycle permeate gas from said subsequent separators, and

means for returning at least part of said recycle permeate gas to said

0110858

means for providing a mixture.

14. The apparatus of claim 13 wherein said first separators have a determined first-separator, membrane surface area and said subsequent separators have a determined subsequent-separator, membrane surface area, the ratio of said first-separator membrane surface are to said subsequent-separator membrane surface area being at least 0.3.

0110858

FIG.I.

FIG.2.

0110858

FIG. 3.

FIG. 4.

# 0110858

European Patent Office

**EUROPEAN SEARCH REPORT**

Application number

EP 83 87 0107

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | FR-A-2 331 368 (TOKYO SHIBAURA ELECTRIC) * Figure 1; page 3, line 10 - page 4, line 7 * | 1,8 | B 01 D 53/22 C 07 C 7/144 |
| A | DE-B-1 167 797 (COMMISSARIAT A L'ENERGIE ATOMIQUE) | | |
| D,A | US-A-4 130 403 (T.E. COOLEY) | | |

|  | TECHNICAL FIELDS SEARCHED (Int. Cl. 3) |
|---|---|
|  | B 01 D 53/00 C 07 C 7/00 C 07 C 9/00 |

The present search report has been drawn up for all claims

| Place of search | Date of completion of the search | Examiner |
|---|---|---|
| THE HAGUE | 31-01-1984 | BOGAERTS M.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82